# EUROPEAN PATENT APPLICATION

(11) **EP 2 954 906 A1**
(43) Date of publication of application: **16.12.2015**
(21) Application number: 13874417.2
(22) Date of filing: 27.12.2013
(51) Int. Cl.: A61L 9/22, A61L 9/015, F24F 3/16, A61L 9/20

(54) **STERILISATION UNIT TO BE INSERTED INTO AN AIR DUCT**

(30) Priority: 08.02.2013 ES 201330150
(71) Applicant: Aero Engineering, S.L., 08290 Cerdanyola del Vallès (Barcelona) (ES)
(72) Inventor: JALDO ROPERO, José Antonio, E-08290 Cerdanyola del Vallès (Barcelona) (ES); BECERRIL RUIZ, José Luis, E-08290 Cerdanyola del Vallès (Barcelona) (ES)
(74) Representative: Oficina Ponti, SLP
(86) International application number: PCT/ES2013/070933
(87) International publication number: WO 2014/122337

(57) **Abstract**

The invention relates to a sterilisation unit intended to be interposed in an air duct, which comprises, arranged successively in a downstream direction, at least one ozone generator (3), a reaction chamber (4) for the reaction of the ozone and the negative ions with the air; and an ozone neutraliser (5), and which further comprises at least one negative ion generator (2) arranged prior to the ozone generator (3).

## Description

The present invention relates to a high-efficiency sterilisation unit intended to be interposed in an air duct.

### Background of the invention

Sterilisation units for interposition in an air duct are well known, comprising, arranged successively in a downstream direction, turbulent regime generation means, at least one ozone generator, a reaction chamber for the reaction of the ozone and the negative ions with the air, and an ozone neutraliser.

An example may be found in that described in the Spanish Utility Model with publication number U1 071 318.

EP2025351 and US20080173178 describe sterilisation devices for closed circuits.

EP 2100624 A1 describes a device equipped with an ioniser and an ozone generator, which gives off ozone.

US2005186108 A1 describes a circuit equipped with ozone reduction means.

None of the devices described is optimal for the sterilisation of air, as they require long routes for the air, which makes them incompatible with their integration into, for example, compact air handling devices.

Specifically:
- They are not adaptable to air conditioning circuits, but are only devices for installation within rooms or enclosed spaces.
- They are very heavy.
- Their mobility and manoeuvrability is difficult.
- Their electrical consumption is high.
- They have a high cost.
- The ionisers used generate ozone externally, which is subsequently inoculated into the chamber, losing oxidant capability along the route and increasing energy consumption.
- If ionisation occurs, this is performed at the outlet, being of use only for the enrichment of the air, and without utilising its sterilisation capability combined with that of the ozone, as will be mentioned below.
- The air is circulated in a low turbulent regime; thus it does not mix appropriately with the ozone, the ions and the disinfection capability is limited to a very low volume of air.
- Throughout the entire route of the air within the channelling duct prior to, during, and subsequent to the phases of the device described, adherence of microbiological material may occur, as well as of dampness which may favour its subsequent development. The sterilisation system acts principally in the air, but traces may remain on the internal surfaces which may develop into resistant colonies which may be drawn by the air and become sites of infection.

On the other hand, in the paper *"*Interaction of ozone and negative air ions to control micro-organisms" by L. Fan, J. Song, P.D. Hildebrand and C.F. Forney, the effect of using ions for the sterilisation of air is described, although said paper does not link this effect with its use in a machine operating in a closed circuit.

Furthermore, the effects of ultraviolet light in the reduction of micro-organisms and the destruction of their DNA are well known. The sterilising properties of plasma are also well known, as is the photocatalytic capability of Titanium Dioxide (TiO₂); that is, the capability to create positive and negative electric charges capable of decomposing the water molecules into hydroxyl radicals which are capable of breaking down organic molecules and destroying them, and also enhancing this capability if the TiO₂, or base material, is doped with materials with high electrical conductivity.

Plasma, in addition to sterilising, also possesses the capability, under certain conditions, to activate the photocatalytic effect when it is directed toward surfaces containing doped titanium dioxide. To this end, there must be a minimum quantity of relative humidity in the air to ensure its effectiveness.

Plasma generators are currently at a stage of development which enables their design in a reduced size, and although they are used in some applications as sterilisers, they are rarely used for sterilisation in air conditioners or in the activation of TiO₂. To achieve this, a Plasma system able to photocatalyse a surface with Titanium Dioxide is required, and with a structure capable of adapting to the various spaces in the ducts of air circuits. A further requirement is a manner of adding the titanium dioxide to the materials and a specific plasma generation so that its combination enables causing a photocatalytic effect. Furthermore, if this sterilising effect caused by photocatalysis could be intensified with other systems which contribute to enhancing the photocatalytic capability, the quality of the air would be improved and the purity of the same would be increased by means of the appropriate combination of various technologies, which would also avoid the close dependence on the need for appropriate relative humidity in the air.

Ultraviolet lamps are usually designed and manufactured in a standard manner, and their use is complicated in some applications and air installations, such as domestic air conditioners, electrical appliance air circuits, heat exchangers, etc. For this purpose, an ultraviolet light system is required which is able to be adapted to different spaces, as is a method for adding the titanium dioxide to the materials whereon it is desired to direct the ultraviolet light in order to bring about the photocatalytic effect.

### Description of the invention

In order to overcome the drawbacks of the state of the art, the present invention proposes a sterilisation unit intended to be interposed in an air duct, comprising, arranged successively in a downstream direction:
- At least one ozone generator:
- At least one means for the generation of negative ions, arranged prior to the ozone generator.
- A reaction chamber for the reaction of the ozone and the negative ions with the air; and
- An ozone neutraliser.

These characteristics resolve drawbacks existing in the state of the art, as:
- The ozone is generated directly within the duct;
- The ions are generated immediately prior to the generation of ozone, causing the negative ions to blend with the ozone, enhancing its oxidant capability, improving the oxidant capability by as much as 43%, as explained in the aforementioned paper.
- Harmful pollutant gases are destroyed.

In accordance with different options, the unit of this invention may comprise one or several of the following features, combined together, if technically possible:
- Means for maximising the turbulent regime prior to the negative ion generator, in order to cause turbulence in the air to enable an improved blending of ozone and air, to thus increase its efficiency.
- A chamber coated with biocidal material arranged prior to, and/or during, and/or subsequent to the means for maximising the turbulent regime. Currently, a series of materials called biocides, whose characteristics enable them to eliminate substances which are harmless to human beings but effective against micro-organisms and which may be released gradually so as to disinfect the surfaces, are known. These solutions, which are usually liquid, may adhere to a material manufactured beforehand by way of an external primer or, depending on the material of which they are manufactured, may be incorporated in the material as an additive, to encapsulate the same within the fabricated item, thus achieving a high efficacy.

- Two symmetrically-arranged ozone generators.
- The ozone neutraliser is a CUO-MnO₂ catalytic-converter honeycomb filter for O₃ to O₂.
- The unit comprises an ozone generator control loop, equipped with an ozone sensor arranged downstream of the ozone neutraliser and connected to the ozone generator.
- The ozone sensor is a nanotube sensor.

On the other hand, the unit may further comprise an additional first sterilisation chamber, equipped with:
- An air inlet.
- A negative and/or positive ion Plasma generator.
- A section partially or totally coated with titanium dioxide with a conductive metal, or a combination of such, which may be Silver, Copper, Iridium, Rhodium, Platinum or other;
- A hydrophilic coating, capable of attracting the humidity present in the atmosphere, condensing it and distributing it evenly over the surface; and
- An air outlet.

In this way, the sterilising effect is enhanced, by means of the formation of a greater number of hydroxyl radicals (OH-) and superoxides (02-), and consequently, also increases the capability of sterilisation.

It is of note that this additional unit represents an invention in itself, and may be employed independently of the ozone unit described above. Preferably, the aforementioned section comprise plasma generators. The chamber may further comprise a matrix of UV LEDs, and a grid, coated with titanium dioxide, may be interposed in the channel.

### Brief description of the drawings

For a better understanding of what has been described above, a set of drawings is attached which, schematically and by way of a non-limiting example only, represent a practical case of embodiment.
Figure 1 is a schematic cross-sectional view of a basic unit in accordance with the invention.
Figure 2 is a plan view of an additional sterilisation chamber featuring plasma generators.
Figure 3 is an elevational cross-section corresponding to the plan view of Figure 2.
Figures 4 and 5 are tubular versions of a chamber with plasma generators.
Figures 6 to 10 are views of sterilisation chambers operating with LEDs.
Figure 11 depicts an embodiment featuring a section with a doped titanium coating, with a hydrophilic surface and with UV ray generators.

### Description of a number of preferred embodiments

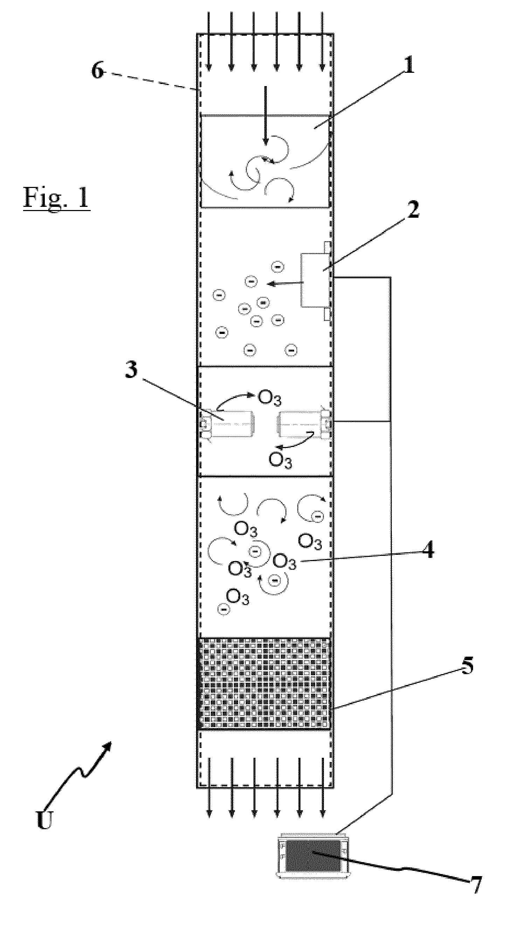

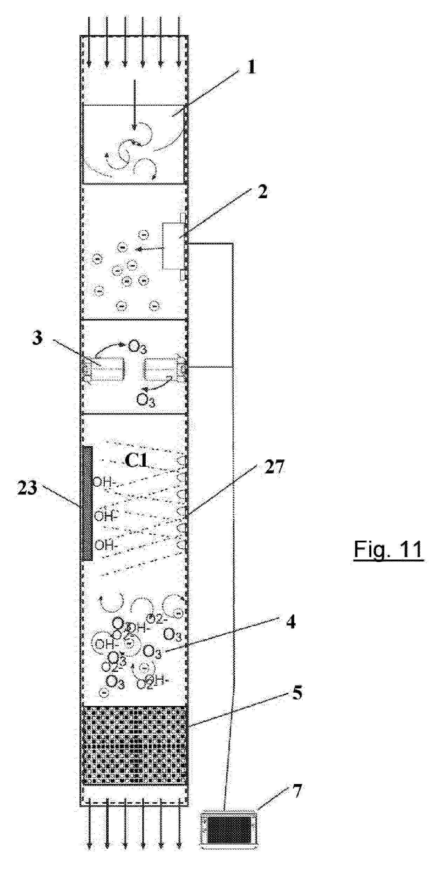

As may be seen in Figures 1 and 11, the invention relates generally to a sterilisation unit U intended to be interposed in an air duct, which comprises, arranged successively in a downstream direction:
- Means for the maximisation of a turbulent regime 1;
- At least one ozone generator 3;
- A reaction chamber 4 for the reaction of the ozone and the negative ions with the air; and
- An ozone neutraliser 5.

These characteristics being well known, for example as in the utility model mentioned in the background of the invention, the present invention is characterised in that it comprises at least one negative ion generator 2 arranged subsequent to the stage of maximisation of the turbulent regime 1 and prior to the ozone generator 3.

Preferably, as may be seen in figure 1, the unit comprises a chamber 6 coated with biocidal material arranged prior to, and/or during, and/or subsequent to the means for maximising the turbulent regime 1.

Moreover, the inventors have been able to verify that the provision of two symmetrically-arranged ozone generators 3 is particularly advantageous.

As an ozone neutraliser 5, a CUO-MnO₂ catalytic-converter honeycomb filter for O₃ to O₂ is preferred.

To ensure that ozone is not expelled into the enclosure whose air it is intended to sterilise, a control loop is foreseen for the ozone generator 3, featuring an ozone sensor 7, preferably made of nanotubes, arranged downstream of the ozone neutraliser 5.

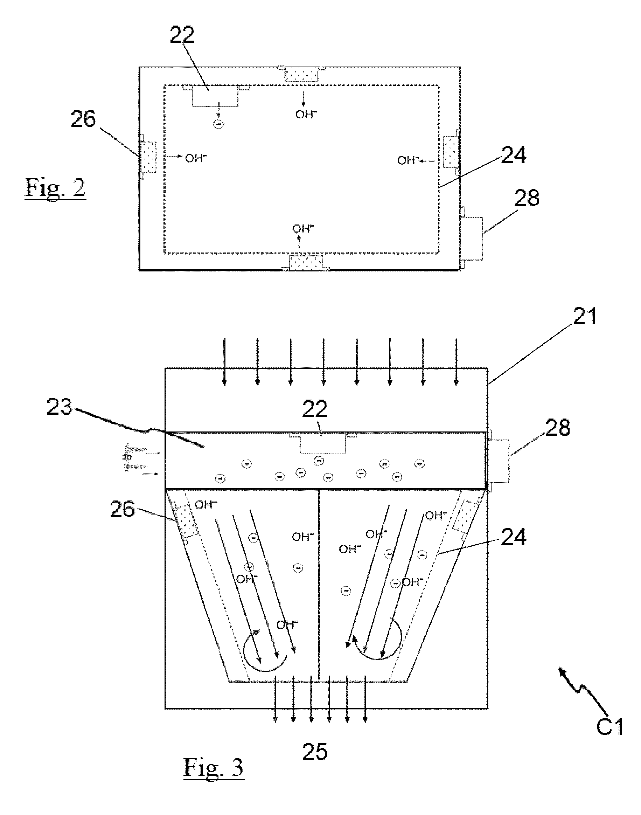

As portrayed in figures 2 and 3, the circuit described may feature a first additional sterilisation chamber C1 equipped with:
- An air inlet 21;
- A negative ion generator 22;
- A section 23 partially or totally coated with titanium dioxide; and
- An air outlet 25.

To enhance the sterilisation still further, said section 23 features plasma generators 26.

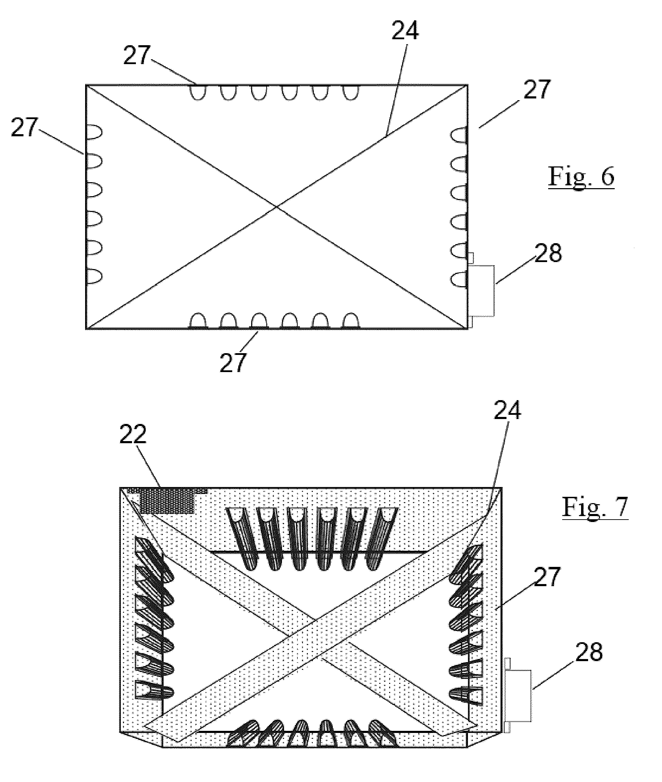

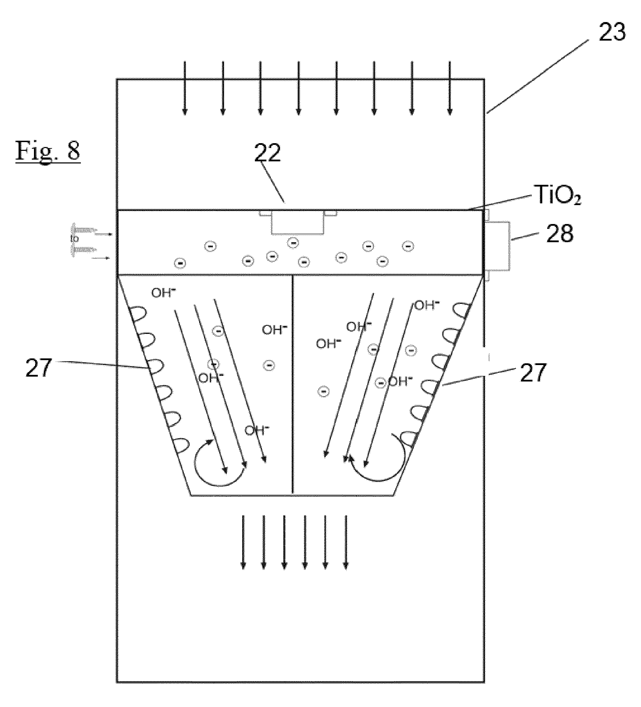

In accordance with another variant, said section 23 comprises a matrix 27 of UV LEDs, as may be observed in figures 6 to 10. Figures 6 to 7 depict arrangements with a rectangular cross-section, while the remainder depict tubular arrangements.

Finally, a grid 24, coated with titanium dioxide, may be interposed in the channel, as may be observed in figures 2 to 9.

### Air sterilisation system with ionised plasma and titanium dioxide

The operation of the chamber with ionised plasma of figures 2 and 3 is described in detail below; this might be claimed independently as it constitutes an invention by itself. Specifically, this invention would consist of a sterilisation chamber C1 provided with an air inlet 21, a plasma or negative ion generator 22, a section 23 partially or totally coated with titanium dioxide, and an air outlet 25.

The system comprises a first stage of negative ionisation. Loading the air with negative ions serves a dual purpose. Ionisation alone has a biocidal effect; furthermore, it will enhance the sterilising effect of the free radicals derived from the photocatalysis caused by the contact between the plasma and the titanium dioxide. This structural arrangement enables the air to be loaded with negative ions at exit, with the well-known consequential advantages of negative ionisation of the air.

The sterilisation system comprises, in a downstream direction, a second phase performed within a container made of metallic or plastic material whose manufacturing base has been performed with a prior blend of titanium dioxide, or with a coating of the same, in addition to a grid 24 located between the plasma generator and the space where the air circulates.

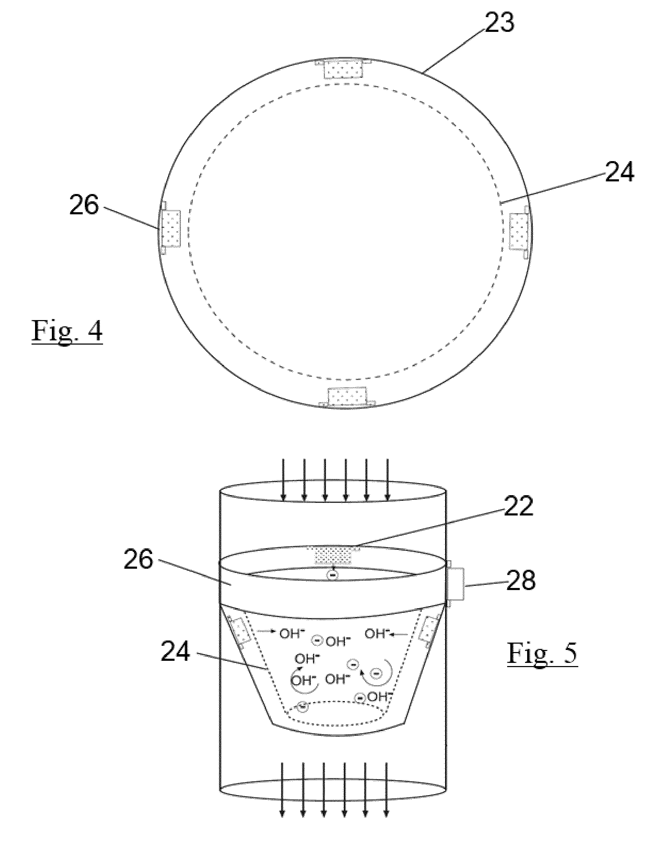

Furthermore, the size of this container diminishes in a downstream direction, causing the concentration of the entire mass of air within a smaller space, thus enabling an enhanced iteration of the sterilising agents with the air to be treated, as may be observed in figures 3 or 5.

This container consists of a first surface which bears the plasma generators, and in front of these, a second grated surface not closed but with perforations, as in a strainer grid, treated with titanium dioxide doped with high-conductivity metals. Contained in the same, or on its surface, a highly hydrophilic material, which will expel the free radicals from the generated photocatalysis into the airstream, is provided. The use of plasma enables the utilisation of this dual capability of sterilisation by plasma and activating the photocatalysis of the titanium dioxide.

The small size of the plasma generators further enables its adaptation to any surface; also achieving a reduction in electrical energy consumption to a great extent, and advantages associated with its durability, weight and cost.

The structure is designed in such a way that all the air passing through the duct is redirected within the container, thus ensuring the passage of the air through this sterilisation phase.

The electrical connection cables are located at the upper part of this container; these will exit to be connected to an electrical transformer located externally to the duct.

### Air sterilisation system by ionised ultraviolet light and titanium dioxide

As may be observed in figures 5 to 9, this other chamber comprises a first phase of negative ionisation. A dual purpose is obtained by loading the air with negative ions. onisation alone has a biocidal effect; furthermore, it will enhance the sterilising effect of the free radicals derived from the photocatalysis without interrupting the path of the ultraviolet light.

This structural disposition enables the air to be loaded with negative ions when it comes out, with the well-known consequential advantages of the negative ionisation of the air.

The sterilisation system comprises, in the direction of the airstream, a second phase comprised of a container made of metallic or plastic material whose manufacturing base has been performed with a prior blend of titanium dioxide doped with metals which increase electronic conductivity, such as Silver, Copper, Rhodium, Iridium or Platinum, or with a coating of the same on the side where ultraviolet light impinges. It further comprises a hydrophilic coating which enables the adherence of the water and its even distribution; this enhancing the generation of hydroxyl OH- free radicals and the sterilising capability.

Figure 11 shows an embodiment provided with section C1, intended to generate a high quantity of ions, particularly O₂⁻ and OH⁻.

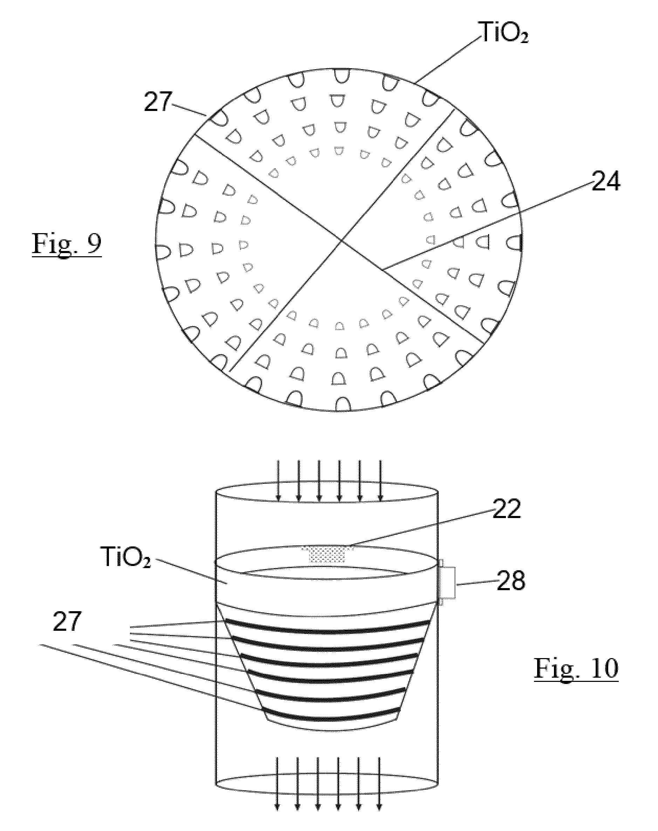

As may be observed for example in figure 9, this container becomes even more reduced in size in the direction of the airstream, causing the concentration of the entire mass of air in a more reduced space, thus enabling an improved iteration of the sterilising agents with the air to be treated.

This container further comprises, on its internal sides, various strips of ultraviolet LED lights. The use of LEDs and not lamps provides an improved adaptability to any of the situations of space and shape which might be required, as their small size and their availability even in prefabricated strips enable their affixing to any surface, also achieving a great reduction in the consumption of electrical energy and the advantages associated with their durability, weight and cost.

The structure is designed in such a way that all the air passing through the duct is redirected through the container, thus ensuring the passage of the air through this sterilisation phase.

The electrical connection cables are located at the upper part of this container; these will exit to be connected to an electrical transformer located externally to the duct.

It should be mentioned that this invention may also be realized in the form of a flexible roll-up tube of airtight fabric or plastic, manufactured with a solution of titanium oxide doped with highly conductive metals, or with a coating of this substance on the side which is in contact with the air.

In addition to the structure of the tube itself, and throughout the length of the same, the ultraviolet LED lights are integrated so as to shine towards the internal section of the tube where the air is to be found, in an arrangement similar to Christmas tree lights, activating the photocatalytic effect of the titanium dioxide.

This distribution may be adapted to smaller air conditioning devices, where the air passes through smaller ducts, being adaptable to any arrangement, by simply replacing the standard air ducts with this sterilising tube.

While the foregoing has been made with reference to particular embodiments of the invention, it will be evident to a person skilled in the art that the sterilisation unit described is susceptible to multiple variations and modifications, and all the details mentioned herein may be replaced by other technically equivalent details without departing from the scope of protection defined by the appended claims.

## Claims

1. A sterilisation unit (U) intended to be interposed in an air duct, which comprises, arranged successively in a downstream direction:
• at least one ozone generator (3);
• a reaction chamber (4) for the reaction of the ozone and the negative ions with the air; and
• an ozone neutraliser (5),
**characterised in that** it comprises at least one negative ion generator (2), arranged prior to the ozone generator (3).

2. A unit as claimed in claim 1, comprising means for the generation of a turbulent regime (1) prior to the ozone generator (3).

3. A unit as claimed in claim 2, comprising a chamber coated with biocidal material (6) arranged prior to the means for the generation of a turbulent regime (1).

4. A unit as claimed in any of the preceding claims, comprising two symmetrically-arranged ozone generators (3).

5. A unit as claimed in any of the preceding claims, wherein the ozone neutraliser (5) is a CUO-MnO₂ catalytic-converter honeycomb filter for O₃ to O₂.

6. A unit as claimed in any of the preceding claims, comprising an ozone generator control loop (3), equipped with an ozone sensor (7) arranged downstream of the ozone neutraliser (5) and connected to the ozone generator (3).

7. A unit as claimed in the preceding claim, wherein the ozone sensor (7) is a nanotube or electrochemical sensor.

8. A unit as claimed in any of the preceding claims, further comprising a first additional sterilisation chamber (C1), equipped with:
• an air inlet (21);
• a negative and/or positive ion generator (22);
• a section (23) partially or totally coated with titanium dioxide; and
• an air outlet (25).

9. A unit as claimed in claim 8, wherein said section (23) comprises plasma generators (26).

10. A unit as claimed in claim 8, wherein said section (23) comprises a matrix of UV LEDs (27).

11. A unit as claimed in claim 10, wherein the UV LEDs (27) are covered by beam-uniformising lenses.

12. A unit as claimed in any of claims 8 to 11, wherein the titanium dioxide coating is doped with Silver, Copper, Rhodium, Iridium or Platinum.

13. A unit as claimed in any of claims 8 to 12, wherein the titanium dioxide coating comprises an additional hydrophilic coating.

14. A unit as claimed in any of claims 8 to 13, comprising a grid (24) interposed within the channel and coated with titanium dioxide.
